(19) Europäisches Patentamt
European Patent Office
Office européen des brevets

(11) Veröffentlichungsnummer: **0 308 740**
**A1**

(12) # EUROPÄISCHE PATENTANMELDUNG

(21) Anmeldenummer: **88114730.0**

(22) Anmeldetag: **09.09.88**

(51) Int. Cl.⁴: **C07D 277/32**

(30) Priorität: **22.09.87 DE 3731803**

(43) Veröffentlichungstag der Anmeldung:
**29.03.89 Patentblatt 89/13**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Diehr, Hans-Joachim, Dr.**
**Höhe 35**
**D-5600 Wuppertal 11(DE)**
Erfinder: **Beck, Gunther, Dr.**
**Am Mittelberg 19**
**D-5090 Leverkusen(DE)**

(54) **Verfahren zur Herstellung von 2,4-Dichlor-5-dichlormethyl-thiazol.**

(57) Man erhält 2,4-Dichlor-5-dichlormethyl-thiazol (I)

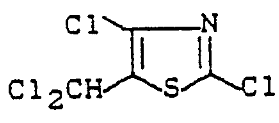

(I)

in hoher Ausbeute und Reinheit, indem man 2,4-Dichlor-5-thiazol-carboxaldehyd (II)

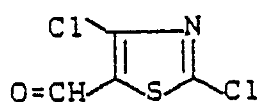

(II)

mit Thionylchlorid ($SOCl_2$) in Gegenwart eines Halogenierungskatalysators bei Temperaturen zwischen 0 °C und 100 °C umsetzt.

(I) kann bekanntermaßen als Bakterizid im Pflanzenschutz und als Zwischenprodukt zur Herstellung von Herbiziden verwendet werden.

EP 0 308 740 A1

## Verfahren zur Herstellung von 2,4-Dichlor-5-dichlormethyl-thiazol

Die Erfindung betrifft ein neues Verfahren zur Herstellung von 2,4-Dichlor-5-dichlormethyl-thiazol.

Es ist bereits bekannt geworden, daß man 2,4-Dichlor-5-dichlormethyl-thiazol erhält, wenn man 2,4-Dichlor-5-methyl-thiazol bei erhöhter Temperatur unter Belichtung mit Chlor umsetzt (vgl. DE-OS 35 05 900 und DE-OS 35 05 902). Hierbei erhält man jedoch immer Produktgemische, welche neben 2,4-Dichlor-5-dichlormethyl-thiazol größere Mengen an 2,4-Dichlor-5-trichlormethyl-thiazol enthalten.

Es ist weiter bekannt, daß man bestimmte aliphatische oder aromatische Aldehyde, wie z. B. 1-Heptanal oder Benzaldehyd, durch Umsetzung mit Phosphorpentachlorid oder mit Thionylchlorid in entsprechende Dichlormethylverbindungen umwandeln kann (vgl J. Am. Chem. Soc. 50 (1928), 172 - 177; J. Org. Chem. 43 (1978), 4367 - 4369; DE-OS 25 25 442). Die Herstellung heterocyclischer Dichlormethylverbindungen aus heterocyclischen Aldehyden ist bisher jedoch nicht bekannt geworden.

Es wurde nun gefunden, daß man 2,4-Dichlor-5-dichlormethyl-thiazol der Formel (I)

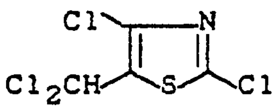

(I)

erhält, wenn man 2,4-Dichlor-5-thiazol-carboxaldehyd der Formel (II)

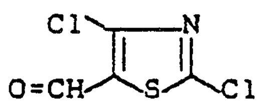

(II)

mit Thionylchlorid ($SOCl_2$) in Gegenwart eines Halogenierungskatalysators bei Temperaturen zwischen 0 °C und 100 °C umsetzt.

Überraschenderweise kann 2,4-Dichlor-5-dichlormethyl-thiazol nach dem erfindungsgemäßen Verfahren in erheblich höherer Ausbeute als nach dem bisher bekannten Herstellungsverfahren erhalten werden.

Vorteile des neuen Verfahrens liegen darin, daß es einfach durchzuführen ist und das Produkt der Formel (I) in so hoher Reinheit liefert, daß eine Reinigung durch Destillation sich erübrigt.

Der Reaktionsablauf beim erfindungsgemäßen Verfahren kann durch das folgende Formelschema skizziert werden:

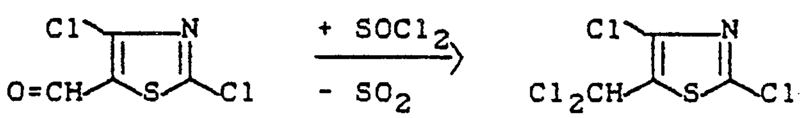


Der beim erfindungsgemäßen Verfahren als Ausgangsstoff zu verwendenden 2,4-Dichlor-5-thiazol-carboxaldehyd der Formel (II) ist bereits bekannt (vgl. DE-OS 33 03 704).

Das erfindungsgemäße Verfahren wird in Gegenwart eines Halogenierungskatalysators durchgeführt. Darunter sind Verbindungen zu verstehen, die gewöhnlich als Katalysatoren bei Reaktionen verwendet werden, bei welchen organisch gebundener Sauerstoff durch Halogen ersetzt wird. Als Halogenierungskatalysatoren werden bevorzugt:

a) aprotische organische Stickstoffverbindungen, wie z. B. Trimethylamin, Triethylamin, Tripropylamin, Tributylamin, N,N-Dimethylanilin, N,N-Diethyl-anilin, N,N-Dimethyl-benzylamin, N,N,-Diethyl-benzylamin, N,N-Dimethyl-cyclohexylamin, Pyridin, 2-Methyl-, 2-Ethyl-, 3-Methyl-, 3-Ethyl-, 4-Methyl- und 4-Ethylpyridin, 2,4-Dimethyl- und 2,6-Dimethyl-pyridin, 2,4,6-Trimethyl-pyridin, 5-Ethyl-2-methyl-pyridin, 1,5-Diazabicyclo-[4,3,0]-non-5-en (DBN), 1,8-Diazabicyclo-[5,4,0]-undec-7-en (DBU), 1,4-Diazabicyclo-[2,2,2]-octan (DABCO), Dimethylformamid, Diethylformamid, Dipropylformamid, Dibutylformamid und Dimethylacetamid,

b) aprotische organische Phosphorverbindungen, wie z. B. Tributylphosphin, Triphenylphosphin, Triscyanethylphosphin, Triphenylphosphindichlorid, Tributylphosphinoxid, Triphenylphosphinoxid und 1-Methyl-1-oxo-phospholin.

Für das erfindungsgemäße Verfahren werden als Halogenierungskatalysatoren Triethylamin, Tripropylamin, Tributylamin, N,N-Dimethyl-benzylamin, 1-Methyl-1-oxo-phospholin und Pyridin besonders bevorzugt eingesetzt.

Die Reaktionstemperaturen können bei dem erfindungsgemäßen Verfahren in einem größeren Bereich variiert werden. Im allgemeinen arbeitet man bei Temperaturen zwischen 0 °C und 100 °C, vorzugsweise bei Temperaturen zwischen 20 °C und 95 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol 2,4-Dichlor-5-thiazol-carboxaldehyd der Formel (II) im allgemeinen 1 bis 10 Mol, vorzugsweise 2 bis 5 Mol, Thionylchlorid und 0,005 bis 0,5 Mol, vorzugsweise 0,01 bis 0,1 Mol, eines Halogenierungskatalysators ein.

Zur Umsetzung können die Reaktionskomponenten in beliebiger Reihenfolge vermischt werden.

In einer bevorzugten Ausführungsform des erfindungsgemäßen Verfahrens werden das Thionylchlorid und der Halogenierungskatalysator vorgelegt und der Aldehyd der Formel (II) wird langsam eindosiert. Das komplette Reaktionsgemisch wird 1 bis 2 Stunden auf 30 °C bis 50 °C erwärmt und dann so lange auf 80 °C bis 95 °C erhitzt, bis keine nennenswerte Gasentwicklung mehr festzustellen ist.

Die Aufarbeitung kann nach üblichen Methoden durchgeführt werden. Vorzugsweise wird das überschüssige Thionylchlorid abdestilliert und der Rückstand mit Wasser und einem mit Wasser praktisch nicht mischbaren organischen Lösungsmittel, wie z. B. Cyclohexan, verrührt oder geschüttelt. Die organische Phase wird abgetrennt, mit Wasser gewaschen und das Lösungsmittel wird abdestilliert. Der verbleibende Rückstand enthält im wesentlichen das Produkt der Formel (I), welches im allgemeinen nicht weiter gereinigt werden muß.

Das nach dem erfindungsgemäßen Verfahren herzustellende 2,4-Dichlor-5-dichlormethyl-thiazol der Formel (I) kann als Bakterizid im Pflanzenschutz verwendet werden oder als Zwischenprodukt zur Herstellung von Herbiziden dienen (vgl. DE-OS 35 05 900).

Herstellungsbeispiele

Beispiel 1

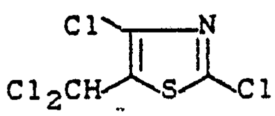

18,2 g (0,1 Mol) 2,4-Dichlor-5-thiazol-carboxaldehyd werden bei 20 °C langsam zu einer Mischung aus 35,7 g (0,3 Mol) Thionylchlorid und 0,5 ml Triethylamin gegeben. Das Reaktionsgemisch wird zunächst eine Stunde lang bei 40 °C bis 45 °C gerührt und dann 3 Stunden auf 90 °C bis 95 °C erhitzt. Das überschüssige Thionylchlorid wird abdestilliert und das zurückbleibende Öl mit je 100 ml Wasser und Cyclohexan versetzt. Die abgetrennte organische Phase wird mit 50 ml Wasser gewaschen und das organische Lösungsmittel wird dann sorgfältig abdestilliert.

Man erhält 2,4-Dichlor-5-dichlormethyl-thiazol als öligen Rückstand. Ausbeute: 24,6 g (98 % der Theorie - bei einem gaschromatographisch bestimmten Gehalt von 94,7 %).

In der nachstehenden Tabelle sind die Resultate analog durchgeführter Umsetzungen unter Variation des Halogenierungskatalysators aufgeführt.

Tabelle

| Resultate weiterer Umsetzungen | | |
|---|---|---|
| Beisp.-Nr. | Halogenierungskatalysator° (eingesetzte Menge) | Ausbeute (% der Theorie) |
| 2 | Dimethylformamid (0,5 ml) | 85 |
| 3 | Pyridin (0,5 ml) | 99 |
| 4 | 4-Dimethylamino-pyridin (0,5 g) | 84 |
| 5 | 1,8-Diazabicyclo-[5,4,0]-undec-7-en (0,5 ml) | 82 |
| 6 | Triphenylphosphinoxid (1 g) | 79 |
| 7 | Tri-n-butylamin (0,5 ml) | 84 |
| 8 | N,N-Dimethyl-benzylamin (0,5 ml) | 95 |
| 9 | 1-Methyl-1-oxo-phospholin (0,5 ml) | 96 |

## Ansprüche

1. Verfahren zur Herstellung von 2,4-Dichlor-5-dichlormethylthiazol der Formel (I)

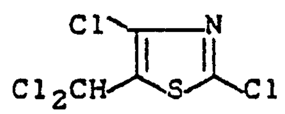

(I)

dadurch gekennzeichnet, daß man 2,4-Dichlor-5-thiazol-carboxaldehyd der Formel (II)

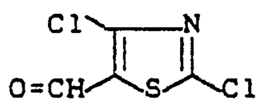

(II)

mit Thionylchlorid ($SOCl_2$) in Gegenwart eines Halogenierungskatalysators bei Temperaturen zwischen 0 °C und 100 °C umsetzt.

2. Verfahren nach Ansprüchen 1, dadurch gekennzeichnet, daß man bei Temperaturen zwischen 20° C und 95° C arbeitet.

3. Verfahren nach Anspruch 1, dadurch gekennzeichnet, daß man auf 1 Mol 2,4-Dichlor-5-thiazolcarboxaldehyd (II) 1-10 Mol Thionylchlorid ($SOCl_2$) und 0,005-0,5 Mol eines Halogenierungskatalysators einsetzt.

4. Verfahren nach Anspruch 1, dadurch gekennzeichnet, daß man auf 1 Mol 2,4-Dichlor-5-thiazolcarboxaldehyd (II) 2-5 Mol Thionylchlorid und 0,01-0,1 Mol eines Halogenierungskatalysators einsetzt.

5. Verfahren nach Anspruch 1, dadurch gekennzeichnet, daß man als Halogenierungskatalysator eine aprotische organische Stickstoffverbindung oder eine aprotische organische Phosphorverbindung einsetzt.

6. Verfahren nach Anspruch 5, dadurch gekennzeichnet, daß man als aprotische organische Stickstoffverbindung eine Verbindung, ausgewählt aus der folgenden Verbindungsgruppe:
Trimethylamin, Triethylamin, Tripropylamin, Tributylamin, N,N-Dimethylanilin, N,N-Diethyl-anilin, N,N-Dimethyl-benzylamin, N,N,m-Diethyl-benzylamin, N,N-Dimethyl-cyclohexylamin, Pyridin, 2-Methyl-, 2-Ethyl-, 3-Methyl-, 3-Ethyl-, 4-Methyl- und 4-Ethyl-pyridin, 2,4-Dimethyl- und 2,6-Dimethyl-pyridin, 2,4,6-Trimethylpyridin, 5-Ethyl-2-methyl-pyridin, 1,5-Diazabicyclo-[4,3,0]-non-5-en (DBN), 1,8-Diazabicyclo-[5,4,0]-undec-7-en (DBU), 1,4-Diazabicyclo-[2,2,2]-octan (DABCO), Dimethylformamid, Diethylformamid, Dipropylformamid, Dibutylformamid und Diethylacetamid,
einsetzt.

7. Verfahren nach Anspruch 5, dadurch gekennzeichnet, daß man als aprotische organische Stickstoffverbindung Triethylamin, Tripropylamin, Tributylamin, N,N-Dimethyl-benzylamin oder Pyridin einsetzt.

8. Verfahren nach Anspruch 5, dadurch gekennzeichnet, daß man als aprotische organische Phosphorverbindung eine Verbindung, ausgewählt aus der folgenden Verbindungsgruppe:
Tributylphosphin, Triphenylphosphin, Triscyanethylphosphin, Triphenylphosphindichlorid, Tributylphosphinoxid, Triphenylphosphinoxid und 1-Methyl -1-oxo-phospholin
einsetzt.

Europäisches Patentamt

# EUROPÄISCHER RECHERCHENBERICHT

Nummer der Anmeldung

EP 88 11 4730

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 192 148 (BAYER AG)<br>* Ansprüche 1,2 *<br>--- | 1-8 | C 07 D 277/32 |
| D,Y | DE-A-2 525 442 (BAYER AG)<br>* Ansprüche *<br>--- | 1-5,8 | |
| D,Y | JOURNAL OF ORGANIC CHEMISTRY, Band 43, Nr. 22, 27. Oktober 1978, American Chemical Society, Washington, DC, US; M.S. NEWMAN et al.: "Conversion of aromatic and alfa,beta-unsaturated aldehydes to dichlorides by thionyl chloride and dimethylformamide"<br>* Insgesamt *<br>----- | 1-6 | |
| | | | RECHERCHIERTE SACHGEBIETE (Int. Cl.4) |
| | | | C 07 D 277/32 |

Der vorliegende Recherchenbericht wurde für alle Patentansprüche erstellt

| Recherchenort | Abschlußdatum der Recherche | Prüfer |
|---|---|---|
| DEN HAAG | 29-11-1988 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)